(19) Europäisches Patentamt
European Patent Office
Office européen des brevets

(11) Veröffentlichungsnummer: **0 337 945**
**A1**

(12) # EUROPÄISCHE PATENTANMELDUNG

(21) Anmeldenummer: **89810253.8**

(22) Anmeldetag: **04.04.89**

(51) Int. Cl.[4]: **C 07 D 487/04**
C 07 D 487/14, C 09 B 57/00,
G 03 G 5/06
//(C07D487/04,209:00,209:00),
(C07D487/14,209:00,209:00,
239:00)

(30) Priorität: **12.04.88 CH 1334/88**

(43) Veröffentlichungstag der Anmeldung:
**18.10.89 Patentblatt 89/42**

(84) Benannte Vertragsstaaten: **DE FR GB IT**

(71) Anmelder: **CIBA-GEIGY AG**
**Klybeckstrasse 141**
**CH-4002 Basel (CH)**

(72) Erfinder: **Rochat, Alain Claude, Dr.**
**Route de Schiffenen 38**
**CA-1700 Fribourg (CH)**

**Iqbal, Abul, Dr.**
**La Dey 202**
**CH-1732 Arconciel (CH)**

**Wallquist, Olof, Dr.**
**Rte. du Confin 31**
**CH-1723 Marly (CH)**

(54) **Monoketo-pyrrolopyrrole.**

(57) Pyrrolopyrrole der Formeln

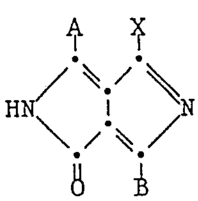

(I)

oder

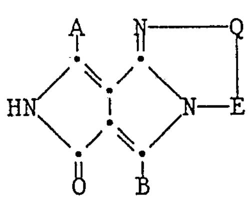

(II),

worin A und B unabhängig voneinander carbocyclische oder heterocyclische aromatische Reste, acyclische oder cyclische aliphatische Reste oder araliphatische Reste sind, Q einen zweiwertigen carbocyclischen oder heterocyclischen aromatischen, acyclischen oder cyclischen aliphatischen oder araliphatischen Rest bedeutet, E eine direkte Bindung oder eine Gruppe -CO- oder -CS- ist, X eine Gruppe -SR oder -NRR′ bedeutet, worin R und R′ unabhängig voneinander Wasserstoff, carbocyclische oder heterocyclische aromatische Reste, acyclische oder cyclische aliphatische Reste oder araliphatische Reste sind und R′ zusätzlich eine Gruppe -Q-Halogen oder -Q-EY-($C_1$-$C_5$-Alkyl), worin Y O oder S bedeutet, sein kann, oder R und R′ mit dem N-Atom, an dem sie gebunden sind, einen 5- oder 6-gliedrigen heterocyclischen Rest bilden.

Pyrrolopyrrole dieser Art eignen sich zum Färben von hochmolekularem organischem Material, wobei farbstarke, brillante und transparente Ausfärbungen mit guten Allgemeinbeständigkeiten erhalten werden, sowie für den Einsatz in photoelektrischen Aufzeichnungsmaterialien als photoleitfähige Substanzen.

EP 0 337 945 A1

**Beschreibung**

## Monoketo-pyrrolopyrrole

Die vorliegende Erfindung betrifft neue 1-Keto-4-thioketo- bzw. 1-Keto-4-mercapto- und 1-Keto-4-amino-pyrrolo[3,4-c]-pyrrole und ihre Verwendung zum Färben von hochmolekularem organischem Material oder als photoleitfähige Substanzen, sowie die Zwischenprodukte zu ihrer Herstellung.

1,4-Diketo-pyrrolo[3,4-c]-pyrrole und ihre Verwendung als Pigmente sind z.B. aus US 4 415 685, US 4 579 949, US 4 720 305, US 4 659 775 und EP-A-232 222 bekannt. 1,4-Dithioketo-pyrrolo[3,4-c]-pyrrole und ihre Eignung als photoleitfähige Substanzen sind beispielsweise in US 4 632 893 und US 4 760 004 beschrieben.

In Angew. Chem. 99 (1987) Nr. 6, 567, beschreiben F. Closs und R. Gompper die Umsetzung von 1,4-Diketo-3,6-diphenyl-pyrrolo[3,4-c]-pyrrol mit Phosphoroxychlorid und Phosphorpentachlorid in Gegenwart von Diethylanilin, wobei einer der beiden Pyrrolringe geöffnet wird.

Es ist nun gefunden worden, dass man eine Ketogruppe von 1,4-Diketo-pyrrolo[3,4-c]-pyrrol überraschenderweise ohne Ringöffnung ersetzen kann, wobei man neue interessante Monoketo-pyrrolopyrrole erhält. Diese neuen Produkte können als Farbmittel zum Färben von hochmolekularem organischem Material, insbesondere von synthetischen Polymeren, bevorzugt von Polyolefinen, Polyestern und Polyamiden, und, vor allem wenn die Ketogruppe durch eine Thioketogruppe ersetzt wird (was bisher nicht möglich war), auch zum Einarbeiten in photoelektrische Aufzeichnungsmaterialien als photoleitfähige Substanzen, eingesetzt werden.

Die vorliegende Erfindung betrifft demnach Pyrrolopyrrole der Formeln

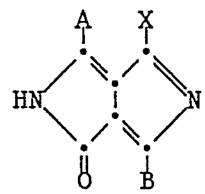

(I)

oder

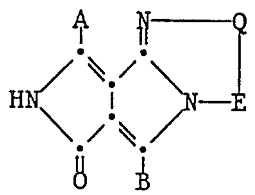

(II),

worin A und B unabhängig voneinander carbocyclische oder heterocyclische aromatische Reste, acyclische oder cyclische aliphatische Reste oder araliphatische Reste sind, Q einen zweiwertigen carbocyclischen oder heterocyclischen aromatischen, acyclischen oder cyclischen aliphatischen oder araliphatischen Rest bedeutet, E eine direkte Bindung oder eine Gruppe -CO- oder -CS- ist, X eine Gruppe -SR oder -NRR′ bedeutet, worin R und R′ unabhängig voneinander Wasserstoff, carbocyclische oder heterocyclische aromatische Reste, acyclische oder cyclische aliphatische Reste oder araliphatische Reste sind und R′ zusätzlich eine Gruppe -Q-Halogen oder -Q-EY-($C_1$-$C_5$-Alkyl), worin Y O oder S bedeutet, sein kann, oder R und R′ mit dem N-Atom an dem sie gebunden sind, einen 5- oder 6-gliedrigen heterocyclischen Rest bilden.

Bedeuten A, B, R und R′ carbocyclische aromatische Reste, dann z.B. mono-, bi-, tri- oder tetracyclische, insbesondere mono- oder bicyclische Reste, wie Phenyl, Diphenyl oder Naphthyl.

Bedeuten A, B, R und R′ heterocyclische aromatische Reste, dann z.B. mono- bis tricyclische. Diese können rein heterocyclisch sein oder einen heterocyclischen Ring und einen oder mehrere ankondensierte Benzolringe enthalten, wobei sie jeweils sowohl über den heterocyclischen wie über den carbocyclischen Teil gebunden sein können. Beispiele von heterocyclischen aromatischen Resten sind Pyridyl, Pyrimidyl, Pyrazinyl, Triazinyl, Furyl, Pyrrolyl, Thiophenyl, Chinolyl, Cumarinyl, Benzofuryl, Benzimidazolyl, Benzoxazolyl, Dibenzofuryl, Benzothiophenyl, Dibenzothiophenyl, Indolyl, Carbazolyl, Pyrazolyl, Imidazolyl, Oxazolyl, Isoxazolyl, Thiazolyl, Indazolyl, Benzthiazolyl, Pyridazinyl, Chinolinyl, Chinazolyl, Chinoxalyl, Phthalazinyl, Phthalazindionyl, Phthalamidyl, Chromonyl, Naphtholactamyl, Chinolonyl, ortho-Sulfobenzoesäureimidyl, Maleinimidyl, Naphtharidinyl, Benzimidazolonyl, Benzoxazolonyl, Benzthiazolonyl, Benzthiazothionyl, Chinazolonyl, Chinoxalonyl, Phthalazonyl, Dioxopyrimidinyl, Pyridonyl, Isochinolonyl, Isochinolinyl, Isothiazolyl, Benzisoxazolyl, Benzisothiazolyl, Indazolonyl, Anthrachinonyl, Acridinyl, Acridonyl, Chinazolindionyl, Chinoxalindionyl, Benzoxazindionyl, Benzoxazinonyl und Naphthalimidyl.

Bedeuten A, B, R und R′ acyclische aliphatische Reste, dann z.B. verzweigte oder unverzweigte, gesättigte oder ungesättigte aliphatische Reste, insbesondere $C_1$-$C_{18}$-Alkylgruppen, wie Methyl, Ethyl, n-Propyl, Isopropyl, n-Butyl, sec.-Butyl, tert.-Butyl, tert.-Amyl, n-Pentyl, n-Hexyl, n-Octyl, 1,1,3,3-Tetramethylbutyl,

Nonyl, Decyl, Undecyl, Dodecyl, Tetradecyl, Hexadecyl und Octadecyl, oder $C_2$-$C_{12}$-Alkenylgruppen, wie Vinyl, Allyl, 1-Propenyl, 1-Methylvinyl, 2-Butenyl, 2-Pentenyl, 3-Hexenyl, 2-Octenyl und 2-Dodecenyl.

Als cyclische aliphatische Reste bedeuten A, B, R und R′ z.B. $C_5$-$C_6$-Cycloalkyl, wie Cyclopentyl oder insbesondere Cyclohexyl.

Als araliphatische Reste bedeuten A, B, R und R′ zum Beispiel $C_7$-$C_{18}$-Aralkyl und insbesondere 1-Phenylethyl, 1,1-Dimethylbenzyl, 2-Phenylethyl oder vor allem Benzyl.

Sowohl die carbocyclischen als auch die heterocyclischen aromatischen Reste, sowie die araliphatischen Reste können übliche nichtwasserlöslichmachende Substituenten aufweisen, wie z.B.:

1) Halogenatome, beispielsweise Chlor, Brom oder Fluor.

2) Verzweigte oder unverzweigte Alkylgruppen mit vorzugsweise 1 bis 18, insbesondere 1 bis 12, vor allem 1 bis 8 und besonders bevorzugt mit 1 bis 5 C-Atomen. Diese Alkylgruppen können nicht-wasserlöslichmachende Substituenten aufweisen, wie beispielsweise Fluor, Hydroxy, Cyano, -$OCOR_1$, -$OR_2$, -$COOR_1$, -$CONR_2R_3$ oder -$OCONHR_1$, worin $R_1$ Alkyl, unsubstituiertes oder durch Halogen, Alkyl oder -O-Alkyl substituiertes Phenyl oder Naphthyl, $R_2$ und $R_3$ Wasserstoff, unsubstituiertes oder durch Cyano oder Hydroxy substituiertes Alkyl, $C_5$-$C_6$-Cycloalkyl, unsubstituiertes oder durch Halogen, Alkyl oder -O-Alkyl substituiertes Phenyl bedeuten, oder worin $R_2$ und $R_3$ zusammen mit dem H-Atom einen 5-6-gliedrigen Heteroring bilden, wie beispielsweise einen Morpholin-, Piperidin- oder Phthalimidring. Weitere mögliche Substituenten an den Alkylgruppen sind mono- oder dialkylierte Aminogruppen, unsubstituiertes oder durch Halogen, Alkyl oder -O-Alkyl substituiertes Phenyl, Naphthyl oder ferner heterocyclische aromatische Reste, wie z.B. die 2-Thienyl-, 2-Benzoxazolyl-, 2-Benzthiazolyl-, 2-Benzimidazolyl-, 6-Benzimidazolonyl-, 2-, 3-oder 4-Pyridyl-, 2-, 4- oder 6-Chinolylreste.

Enthalten die unter 2) genannten Substituenten ihrerseits wieder Alkyl, so kann dieses Alkyl verzweigt oder unverzweigt sein und vorzugsweise 1 bis 18, insbesondere 1 bis 12, vor allem 1 bis 8 und besonders bevorzugt 1 bis 5 C-Atomen enthalten.

Beispiele von unsubstituierten oder substituierten Alkylgruppen sind Methyl, Ethyl, n-Propyl, Isopropyl, n-Butyl, sec.-Butyl, tert.-Butyl, tert.-Amyl, n-Pentyl, n-Hexyl, 1,1,3,3-Tetramethylbutyl, n-Heptyl, n-Octyl, Nonyl, Decyl, Undecyl, Dodecyl, Hydroxymethyl, Trifluormethyl, Trifluorethyl, Cyanmethyl, Methoxycarbonylmethyl, Acetoxymethyl oder Benzyl.

3) Die Cyangruppe.

4) Die Gruppe der Formel -$NR_2R_3$, worin $R_2$ und $R_3$ die unter 2) angegebene Bedeutung haben. Als Beispiele seien genannt: Amino, Methylamino, Dimethylamino, Ethylamino, Diethylamino, Isopropylamino, β-Hydroxyethylamino, β-Hydroxypropylamino, N,N-Bis-(β-hydroxyethyl)-amino, N,N-Bis-(β-cyanethyl)-amino, Cyclohexylamino, Phenylamino, N-Methylphenylamino, Benzylamino, Dibenzylamino, Piperidyl, 2,2,6,6-Tetramethylpiperid-1-yl oder -4-yl oder Morpholyl.

5) Die Gruppe der Formel -$CYOR_1$, worin Y O oder S ist und $R_1$ die unter 2) angegebene Bedeutung hat. Als Beispiele für $R_1$ seinen genannt: Methyl, Ethyl, Isopropyl, tert.-Butyl, n-Butyl, t-Amyl, Phenyl, Benzyl, Furfuryl, 2-(Dimethylamino)-ethyl oder 2-Cyanoethyl.

6) Die Gruppe -$OR_4$, worin $R_4$ Wasserstoff, Alkyl, Aryl, beispielsweise Naphthyl oder insbesondere unsubstituiertes oder durch Halogen, Alkyl, oder -O-Alkyl substituiertes Phenyl, $C_5$-$C_6$-Cycloalkyl, Aralkyl oder einen heterocyclischen Rest bedeutet. In den Definitionen von $R_4$ vorkommendes Alkyl kann z.B. eine der unter 2) als bevorzugt angegebenen Anzahl C-Atome haben. Als Beispiele von $R_4$ seien genannt: Methyl, Ethyl, n-Propyl, Isopropyl, Trifluorethyl, Phenyl, o-, m- oder p-Chlorphenyl, o-, m- oder p-Methylphenyl, α- oder β-Naphthyl, Cyclohexyl, Benzyl, Thienyl oder Pyranylmethyl.

7) Die Gruppe -$SR_4$, worin $R_4$ die unter 6) angegebene Bedeutung hat. Als Beispiele für $R_4$ seien genannt: Methyl, Ethyl, n-Propyl, Isopropyl, Phenyl, o-, m- oder p-Chlorphenyl, o-, m- oder p-Methylphenyl, α- oder β-Naphthyl, Cyclohexyl, Benzyl, Thienyl oder Pyranylmethyl.

8) Die Gruppe der Formel -$CYR_4$, worin Y O oder S ist und $R_4$ die unter 6) angegebene Bedeutung hat. Als Beispiele für $R_4$ seien genannt: Methyl, Ethyl, tert.-Butyl, Phenyl, o-, m- oder p-Chlorphenyl, o-, m- oder p-Methylphenyl, oder α- bzw. β-Naphthyl, 2-Cyanethyl oder 2-Mercaptoethyl.

9) Die Gruppe der Formel -$NR_5CYR_1$, worin Y O oder S ist, $R_1$ die unter 2) angegebene Bedeutung hat, $R_5$ Wasserstoff, Alkyl, Aryl, beispielsweise Naphthyl oder insbesondere unsubstituiertes oder durch Halogen, Alkyl oder -O-Alkyl substituiertes Phenyl, $C_5$-$C_6$-Cycloalkyl, Aralkyl oder den Rest -$COR_1$ bedeutet, wobei zwei Reste -$COR_1$ zusammen mit dem N-Atom einen heterocyclischen Ring bilden können. In den Definitionen von $R_5$ vorkommendes Alkyl kann z.B. eine der unter 2) als bevorzugt angegebenen Anzahl C-Atome haben. Als Beispiele seien genannt: Acetylamino, Thioacetylamino, Propionylamino, Butyrylamino, Benzoylamino, Thiobenzoylamino, p-Chlorbenzoylamino, p-Methylbenzoylamino, N-Methylacetylamino, N-Methyl-benzoylamino, N-Succinimido oder N-Phthalimido.

10) Die Gruppe der Formel -$NR_4COOR_1$, worin $R_1$ und $R_4$ die unter 2) bzw. 6) angegebene Bedeutung haben. Als Beispiele seien die Gruppen -$NHCOOCH_3$, -$NHCOOC_2H_5$ oder -$NHCOOC_6H_5$ genannt.

11) Die Gruppe der Formel -$NR_4CYNR_2R_3$, worin Y O oder S ist, $R_4$, $R_2$ und $R_3$ die unter 6) bzw. 2) angegebene Bedeutung haben. Als Beispiele seien genannt: Ureido, N-Methylureido, N-Phenylureido oder N, N-2′,4′-Dimethylphenylureido, sowie deren Thiocarbonyl-Analoge.

12) Die Gruppe der Formel -$NR_5SO_2R_1$, worin $R_1$ die unter 2) und $R_5$ die unter 9) angegebene Bedeutung haben. Als Beispiele seien genannt: Methansulfonylamino, Phenylsulfonylamino, p-Toluylsulfonylamino oder β-Naphthylsulfonylamino.

13) Die Gruppen der Formel $-SO_2R_1$ oder $-SOR_1$, worin $R_1$ die unter 2) angegebene Bedeutung hat. Als Beispiele seien genannt: Methylsulfonyl, Ethylsulfonyl, Phenylsulfonyl, 2-Naphthylsulfonyl, Phenylsulfoxidyl.

14) Die Gruppe der Formel $-SO_2OR_1$, worin $R_1$ die unter 2) angegebene Bedeutung hat. Als Beispiele für $R_1$ seien genannt: Methyl, Ethyl, Phenyl, o-, m- oder p-Chlorphenyl, o-, m- oder p-Methylphenyl, α-oder β-Naphthyl.

15) Die Gruppe der Formel $-CYNR_2R_3$, worin Y O oder S ist und $R_2$ und $R_3$ die unter 2) angegebene Bedeutung haben. Als Beispiele seien genannt: Carbamoyl, N-Methylcarbamoyl, N-Ethylcarbamoyl, N-Phenylcarbamoyl, N,N-Dimethyl-carbamoyl, N-Methyl-N-phenylcarbamoyl, N-α-Naphthylcarbamoyl, oder N-Piperidylcarbamoyl, sowie deren Thiocarbonyl-Analoge.

16) Die Gruppe der Formel $-SO_2NR_2R_3$, worin $R_2$ und $R_3$ die unter 2) angegebene Bedeutung haben. Als Beispiele seien genannt: Sulfamoyl, N-Methylsulfamoyl, N-Ethylsulfamoyl, N-Phenylsulfamoyl, N-Methyl-N-phenylsulfamoyl oder N-Morpholylsulfamoyl.

17) Die Gruppe der Formel $-N=N-R_6$, worin $R_6$ den Rest einer Kupplungskomponente oder einen unsubstituierten oder durch Halogen, Alkyl oder -O-Alkyl substituierten Phenylrest bedeutet. In den Definitionen von $R_6$ vorkommendes Alkyl kann z.B. eine der unter 2) als bevorzugt angegebenen Anzahl C-Atome haben. Als Beispiele für $R_6$ seien genannt: die Acetoacetarylid-, Pyrazolonyl-, Pyridonyl-, o- oder p-Hydroxyphenyl-, o-Hydroxynaphthyl-, p-Aminophenyl- oder p-N,N-Dimethylaminophenylreste.

18) Die Gruppe der Formel $-OCOR_1$, worin $R_1$ die unter 2) angegebene Bedeutung hat. Als Beispiele für $R_1$ seien genannt: Methyl, Ethyl, Phenyl, o-, m- oder p-Chlorphenyl, 2-, 3- oder 4-Pyridyl.

19) Die Gruppe der Formel $-OCONHR_1$, worin $R_1$ die unter 2) angegebene Bedeutung hat. Als Beispiele für $R_1$ seien genannt: Methyl, Ethyl, Phenyl, o-, m- oder p-Chlorphenyl.

Die acyclischen und cyclischen aliphatischen Reste können auch übliche nichtwasserlöslichmachende Substituenten aufweisen, wie z.B. diejenigen, die unter den Punkten 1, 3 bis 16, 18 und 19 aufgeführt werden.

Q ist in Verbindungen der Formel II, wenn E -CO- oder -CS- bedeutet, bevorzugt eine Gruppe der Formeln

$-C(R_7)(R_8)-$, (III) $-C(R_9)(R_{10})-C(R_{11})(R_{12})-$ (IV) oder $-CR_{13}=CR_{14}-$ (V)

und, wenn E eine direkte Bindung bedeutet, bevorzugt eine Gruppe der Formeln IV oder V, wobei in den Formeln III bis V $R_7$ und $R_8$ unabhängig voneinander Wasserstoff, $C_1$-$C_5$-Alkyl, $C_5$-$C_8$-Cycloalkyl, unsubstituiertes oder durch Halogen, Hydroxy, $C_1$-$C_5$-Alkyl oder $C_1$-$C_5$-Alkoxy oder Phenoxy substituiertes Phenyl oder Benzyl oder eine der Gruppen

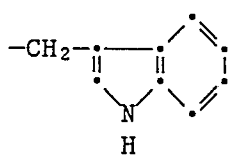
,
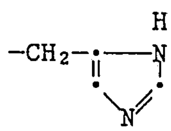

oder $-(CH_2)_2-S(C_1$-$C_5$-Alkyl) bedeuten,

$R_9$, $R_{10}$, $R_{11}$ und $R_{12}$ unabhängig voneinander Wasserstoff, $C_1$-$C_5$-Alkyl, $C_5$-$C_6$-Cycloalkyl, unsubstituiertes oder durch Halogen, Hydroxy, $C_1$-$C_5$-Alkyl oder $C_1$-$C_5$-Alkoxy substituiertes Phenyl oder Benzyl bedeuten oder $R_{10}$ und $R_{12}$ zusammen mit den C-Atomen, an denen sie gebunden sind, eine $C_5$-$C_6$-Cycloalkylengruppe bilden, und $R_{13}$ und $R_{14}$ unabhängig voneinander Wasserstoff, $C_1$-$C_5$-Alkyl, $C_5$-$C_6$-Cycloalkyl, unsubstituiertes oder durch Halogen, Hydroxy, $C_1$-$C_5$-Alkyl oder $C_1$-$C_5$-Alkoxy substituiertes Phenyl oder Benzyl bedeuten, oder $R_{13}$ und $R_{14}$ zusammen mit den C-Atomen, an denen sie gebunden sind, einen Naphthalin-, Anthracen- oder Phenanthrenring oder eine der Gruppen

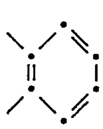
,
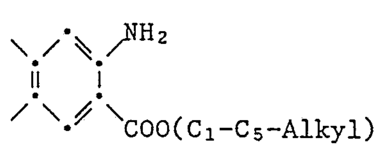
,
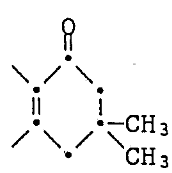
,
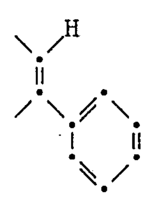
oder

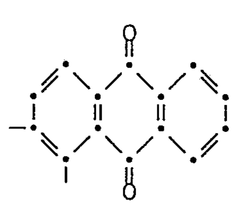
bilden.

$R_7$, $R_9$ und $R_{11}$ sind bevorzugt Wasserstoff. $R_{13}$ und $R_{14}$ sind bevorzugt Wasserstoff oder sie bilden

zusammen mit den C-Atomen, an denen sie gebunden sind, vorzugsweise eine Gruppe

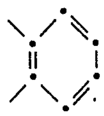 .

Beispiele für Reste Q der Formel III sind

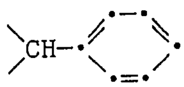 , 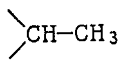 , 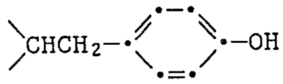 , 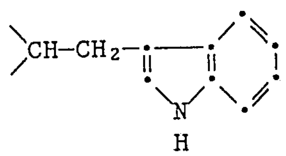

und $>CH-(CH_2)_2-SCH_3$.

Beispiele für Reste Q der Formel IV sind

$-CH_2-CH_2-$, 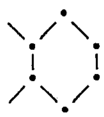 , 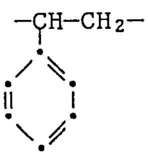 und $-CH(CH_3)-CH_2-$.

Beispiele für Reste Q der Formel V sind

$-CH=CH-$, 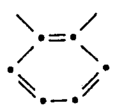 , 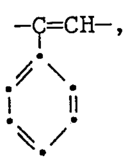 $-C(CH_3)=CH-$, 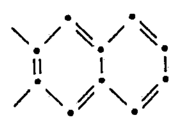 ,

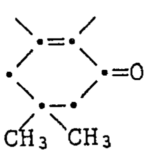 , 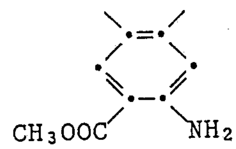 und 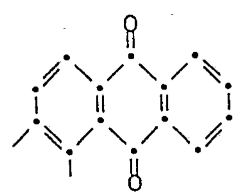 .

Bedeutet X in der Verbindung der Formel I eine Gruppe SH oder NHR′ (d.h. wenn R Wasserstoff bedeutet), so entspricht die Strukturformel I jeweils nur einer ihrer möglichen tautomeren Formen. Diese Verbindungen liegen allerdings auch, und in vielen Fällen sogar bevorzugt, in der in den folgenden Formeln dargestellten Form vor:

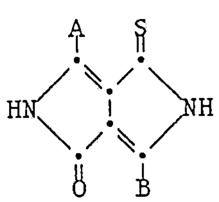 und 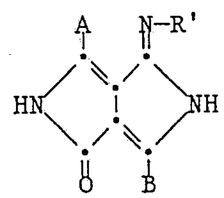 ,

worin A, B und R′ die oben angegebene Bedeutung haben.

Von besonderem Interesse sind Pyrrolopyrrole der Formeln I oder II, worin A und B unabhängig voneinander eine der Gruppen

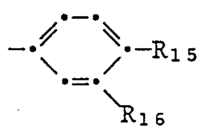
oder
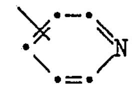

bedeuten, worin $R_{15}$ und $R_{16}$ unabhängig voneinander Wasserstoff, Halogen, Nitro, Carbamoyl, Cyano, Trifluormethyl, $C_2$-$C_{13}$-Alkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxycarbonyl, $C_2$-$C_{13}$-Alkanoylamino, $C_1$-$C_{12}$-Monoalkylamino, $C_2$-$C_{24}$-Dialkylamino, Mono-($C_5$-$C_7$-cycloalkyl)-amino, Di-($C_5$-$C_7$-cycloalkyl)-amino, N-Pyrrolidino, N-Piperidino, N-Morpholino, unsubstituiertes oder durch Halogen, $C_1$-$C_{12}$-Alkyl oder $C_1$-$C_{12}$-Alkoxy substituiertes Phenyl, Phenoxy, Phenylmercapto, Phenoxycarbonyl oder Phenylcarbamoyl sind,
X eine Gruppe -SR oder -NRR′ bedeutet,
R Wasserstoff, $C_1$-$C_{12}$-Alkyl, Cyclohexyl, β-Methoxyallyl, Benzyl, unsubstituiertes oder durch Halogen, Cyano, Nitro, $C_1$-$C_5$-Alkyl, $C_1$-$C_5$-Alkoxy, ($C_1$-$C_5$-Alkoxy)-oarbonyl, $C_1$-$C_5$-Alkylmercapto, Phenylmercapto oder Phenoxy substituiertes Phenyl ist, oder R ferner Pyridyl, Pyrimidyl, Pyrazinyl, Triazinyl, Furyl, Pyrrolyl, Thienyl, Chinolinyl, Cumarinyl, Benzofuryl, Benzimidazolyl oder Benzoxazolyl bedeutet,
R′ $C_1$-$C_{12}$-Alkyl, β-Dimethylaminoethyl, Cyclohexyl, Cyanethyl, β-Ethoxycarbonylethyl, Benzyl, 1-Phenyl-2-ethoxycarbonyl-vinyl, unsubstituiertes oder durch Halogen, Cyano, Nitro, $C_1$-$C_5$-Alkyl, $C_1$-$C_5$-Alkoxy, ($C_1$-$C_5$-Alkoxy)-carbonyl, $C_1$-$C_5$-Alkylmercapto, Phenoxy oder Phenylmercapto substituiertes Phenyl ist, oder R′ ferner Pyridyl, Pyrimidyl, Chinolinyl, Pyrazinyl, Triazinyl, Furyl, Pyrrolyl, Thienyl, Cumarinyl, Benzofuryl, Anthrachinonyl, Benzimidazolyl oder Benzoxazolyl ist, oder eine Gruppe
-Q-EY-($C_1$-$C_5$-Alkyl),
bedeutet, oder R und R′ zusammen mit dem N-Atom einen Piperidin-, 2,2,6,6-Tetramethylpiperidin-, Pyrrol-, Morpholin-, Pyrrolidin- oder Phthalimidring bilden,
E und Y die oben angegebene Bedeutung haben, und
Q unsubstituiertes oder durch Halogen, Cyano, Nitro, $C_1$-$C_5$-Alkyl, $C_1$-$C_5$-Alkoxy oder ($C_1$-$C_5$-Alkoxy)-carbonyl substituiertes o-Phenylen oder eine zweiwertige Gruppe der Formel
$-C(R_7)(R_8)-$ oder $-CR_{13}=CR_{14}-$ bedeutet,
$R_7$ und $R_8$ unabhängig voneinander Wasserstoff, $C_1$-$C_5$-Alkyl oder Phenyl sind und
$R_{13}$ und $R_{14}$ unabhängig voneinander Wasserstoff, $C_1$-$C_5$-Alkyl oder Phenyl sind oder zusammen mit den C-Atomen an denen sie gebunden sind, einen Naphthalin-, Anthracen- oder Phenanthrenring bilden.

Beispiele von $C_1$-$C_5$-Alkylgruppen sind Methyl, Ethyl, n-Propyl, Isopropyl, n-Butyl, sec.-Butyl, tert.-Butyl, n-Pentyl und tert.-Amyl, und von $C_1$-$C_{12}$-Alkylgruppen zusätzlich z.B. noch n-Hexyl, 1,1,3,3-Tetramethylbutyl, n-Heptyl, n-Octyl, Nonyl, Decyl, Undecyl und Dodecyl.

Beispiele von $C_5$-$C_6$ Cycloalkylgruppen sind Cyclopentyl und insbesondere Cyclohexyl.

$C_1$-$C_5$-Alkoxygruppen sind z.B.: Methoxy, Ethoxy, Propoxy, Isopropoxy, n-Butoxy, sec.-Butoxy, tert.-Butoxy, n-Pentyloxy and tert.-Amyloxy und $C_1$-$C_{12}$-Alkoxygruppen zusätzlich z.B. noch n-Hexyloxy, n-Heptyloxy, n-Octyloxy, Decyloxy und Dodecyloxy.

$C_1$-$C_5$-Alkylmercapto bedeutet z.B. Methylmercapto, Ethylmercapto, Propylmercapto, Isopropylmercapto, n-Butylmercapto, sec.-Butylmercapto, tert.-Butylmercapto und tert.-Amylmercapto.

Beispiele von $C_2$-$C_{13}$-Alkylcarbamoylgruppen sind N-Methylcarbamoyl, N-Ethylcarbamoyl, N,N-Dimethylcarbamoyl, N-Butylcarbamoyl, N,N-Dibutylcarbamoyl und N,N-Dihexylcarbamoyl.

$C_1$-$C_{12}$-Alkylmercapto bedeutet z.B. Methylmercapto, Ethylmercapto, Propylmercapto, Butylmercapto, n-Pentylmercapto, tert.-Amylmercapto, n-Hexylmercapto, Decylmercapto und Dodecylmercapto.

$C_2$-$C_{13}$-Alkoxycarbonylgruppen stellen z.B. Methoxy-, Ethoxy-, Propoxy-, Isopropoxy-, n-Butoxy-, tert.-Butoxy-, n-Hexyloxy-, n-Octyloxy- und Dodecyloxy-carbonyl dar.

$C_2$-$C_{13}$-Alkanoylamino ist z.B. Acetylamino, Propionylamino, Butyrylamino.

$C_1$-$C_{12}$-Monoalkylamino bedeutet z.B. Methylamino, Ethylamino, Isopropylamino, n-Butylamino, n-Octylamino und n-Decylamino.

Mono-($C_5$-$C_7$-cycloalkyl)-amino bedeutet z.B. Cyclohexylamino.

$C_1$-$C_{12}$-Dialkylamino bedeutet z.B. Dimethylamino, Diethylamino, Dibutylamino und Dihexylamino.

Di-($C_5$-$C_7$-cycloalkyl)-amino bedeutet z.B. Dicyclohexylamino.

Halogen ist z.B. Fluor, Brom und insbesondere Chlor.

Bevorzugt sind Pyrrolopyrrole der Formeln I oder II, worin A und B gleich sind und eine der Gruppen

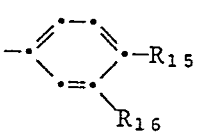
oder
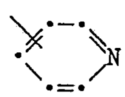

bedeuten, worin einer der Substituenten $R_{15}$ und $R_{16}$ ein Wasserstoff-, Chlor- oder Bromatom, eine Methyl-, Methoxy-, Methylmercapto-, Cyan-, Phenyl-, Phenoxy- oder Phenylmercaptogruppe ist und der andere ein Wasserstoffatom bedeutet,
X eine Gruppe -SH oder -NHR′ bedeutet,
R′ $C_1$-$C_{12}$-Alkyl, unsubstituiertes oder durch Halogen, Cyano, Nitro, Methyl, Methoxy, Methylmercapto,

Methoxy- oder Ethoxycarbonyl substituiertes Phenyl ist oder eine Gruppe -Q-EY($C_1$-$C_5$-Alkyl) bedeutet, E und Y die oben angegebene Bedeutungen haben und Q o-Phenylen oder eine zweiwertige Gruppe der Formel -$CHR_8$- oder -$CR_{13}=CR_{14}$- bedeutet, $R_8$ Wasserstoff, Methyl oder Phenyl bedeutet und $R_{13}$ und $R_{14}$ Wasserstoff, Methyl oder Phenyl sind.

Besonders bevorzugt werden Pyrrolopyrrole der Formeln I oder II, worin A und B gleich sind und eine Gruppe

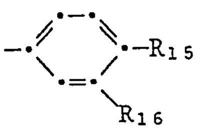

bedeuten, worin einer der Substituenten $R_{15}$ und $R_{16}$ ein Wasserstoff- oder Chloratom, eine Methyl-, Cyan- oder Phenylgruppe ist und der andere ein Wasserstoffatom bedeutet, X eine Gruppe -SH oder -NHR′ bedeutet, R′ unsubstituiertes oder durch Chlor, Brom, Methyl, Cyano, Nitro, Methoxy, Methylmercapto, Methoxy- oder Ethoxycarbonyl substituiertes Phenyl ist, E die oben angegebene Bedeutung hat und Q o-Phenylen bedeutet.

Die Herstellung der Pyrrolopyrrole der Formeln I und II kann z.B. nach folgenden Verfahren erfolgen:

a) Durch Umsetzung eines Pyrrolopyrrols der Formel

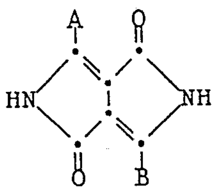

(VI),

worin A und B die oben angegebene Bedeutung haben, mit einer Verbindung der Formel DG (VII), worin D Chlor oder Brom ist und G eine Gruppe -SOCl, -SOBr, -$POCl_2$, -POClBr, -$POBr_2$, -POClZ oder -POBrZ bedeutet und Z Phenyl, Phenoxy, Phenylmercapto, Di-($C_1$-$C_4$-alkyl)-amino, Phenylamino, Diphenylamino oder Phenyl-($C_1$-$C_4$-alkyl)-amino ist, wobei die Phenylgruppen auch durch Halogen oder $C_1$-$C_5$-Alkyl substituiert sein können, in Gegenwart eines geeigneten Katalysators, bevorzugt eines sekundären Amids, wie z.B. Dimethylformamid, Dimethylacetamid oder N-Methylpyrrolidon, erhält man ein Pyrrolopyrrol der Formel

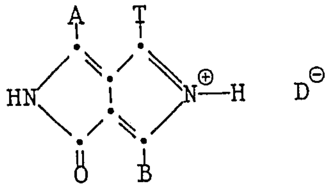

(VIII),

worin A und B die oben angegebene Bedeutung haben und T Chlor, Brom oder eine Gruppe -O-G bedeutet, worin G die oben angegebene Bedeutung hat.

D ist bevorzugt Chlor und G ist bevorzugt eine Gruppe -POClZ, worin Z Phenyl, Phenoxy, Dimethylamino oder Phenylamino bedeutet, oder insbesondere eine Gruppe -$POCl_2$.

b) Durch Umsetzung des Zwischenproduktes der Formel VIII nach an sich bekannten Methoden mit einer Verbindung der Formel

HSR (IX)

oder HNRR′ (X),

worin R und R′ die oben angegebene Bedeutung haben, oder mit Salzen der Verbindungen der Formeln IX oder X, erhält man nach dem Neutralisieren, um die Isolierung eines Halohydrates zu vermeiden, die entsprechende Verbindung der Formel I.

c) Erwärmt man Pyrrolopyrrole der Formel I, worin X eine Gruppe

-NH-Q-Halogen oder

-NH-Q-EY-($C_1$-$C_5$-Alkyl)

bedeutet, in einem geeigneten organischen Lösungsmittel, gegebenenfalls in Gegenwart eines geeigneten Katalysators, nach allgemein bekannten Cyclisierungsmethoden, so erhält man durch

Cyclisierung die entsprechende Verbindung der Formel II.

Bei den Verbindungen der Formeln VI, VII, IX und X handelt es sich um bekannte Verbindungen. Sollten einige noch neu sein, so können sie in Analogie zu allgemein bekannten Methoden hergestellt werden.

Verwendet man für den Schritt b) Salze der Verbindungen der Formeln IX oder X, so handelt es sich zweckmässig bei den Verbindungen der Formel IX z.B. um deren Alkalimetallsalze und bei den Verbindungen der Formel X z.B um deren Chlorhydrate.

Geeignete Lösungsmittel für den Schritt b) sind beispielsweise Wasser oder wasserfreie organische Lösungsmittel, die gegenüber den Verbindungen der Formel VIII inert sind, wie z.B. die halogenierten aliphatischen Kohlenwasserstoffe Dichlorethan, Tetrachlorethan, Chloroform oder Tetrachlorkohlenstoff oder insbesondere das unter dem Namen Sulfolan bekannte Tetrahydrothiophen-1,1-dioxyd.

Bei der Umsetzung mit Verbindungen der Formel IX, worin R Wasserstoff ist, empfiehlt es sich, die entsprechenden Alkalisalze, wie z.B. Natrium-oder Kaliumsulfid, einzusetzen.

Bei Cyclisierungen gemäss Schritt c) werden zweckmässig geeignete Katalysatoren eingesetzt. Bei Cyclisierungen von Verbindungen der Formel I, worin X eine Gruppe

-NH-Q-Halogen

bedeutet, eignen sich z.B. metallisches Kupfer oder Kupferhalogenide oder Gemische davon. Bei Cyclisierungen von Verbindungen der Formel I, worin X eine Gruppe

-NH-Q-EY-($C_1$-$C_5$-Alkyl)

bedeutet, eignen sich insbesondere saure Katalysatoren, wie z.B. Eisessig oder p-Toluolsulfonsäure.

Die oben beschriebene Herstellung von Verbindungen der Formel I kann sowohl stufenweise, mit oder ohne Isolierung des Zwischenproduktes der Formel VIII, oder im Eintopfverfahren durchgeführt werden. Für das Eintopfverfahren hat sich Sulfolan als geeignetstes Lösungsmittel erwiesen.

Die als Zwischenprodukte entstehenden Pyrrolopyrrole der Formel

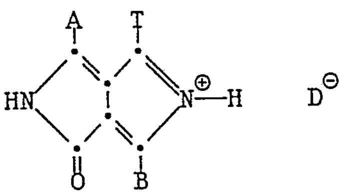 (VIII),

worin A und B unabhängig voneinander carbocyclische oder heterocyclische aromatische Reste, acyclische oder cyclische aliphatische Reste oder araliphatische Reste sind, und T Chlor, Brom oder eine Gruppe -O-G bedeutet, worin G eine Gruppe -SOCl, -SOBr, -$POCl_2$, -POClBr, -$POBr_2$, -POClZ oder -POBrZ bedeutet und Z Phenyl, Phenoxy, Phenylmercapto, Di-($C_1$-$C_4$-alkyl)-amino, Phenylamino, Diphenylamino oder Phenyl-($C_1$-$C_4$-alkyl)-amino ist, wobei die Phenylgruppen auch durch Halogen oder $C_1$-$C_5$-Alkyl substituiert sein können,

sind neu und bilden einen weiteren Gegenstand der vorliegenden Erfindung.

Von besonderem Interesse als Zwischenprodukte sind Pyrrolopyrrole der Formel VIII, worin A und B unabhängig voneinander eine der Gruppen

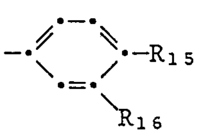 oder 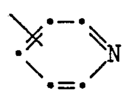

bedeuten, worin $R_{15}$ und $R_{16}$ unabhängig voneinander Wasserstoff, Halogen, Nitro, Carbamoyl, Cyano, Trifluormethyl, $C_2$-$C_{13}$-Alkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxycarbonyl, $C_2$-$C_{13}$-Alkanoylamino, $C_1$-$C_{12}$-Monoalkylamino, $C_2$-$C_{24}$-Dialkylamino, Mono-($C_5$-$C_7$-cycloalkyl)-amino, Di-($C_5$-$C_7$-cycloalkyl)-amino, H-Pyrrolidino, H-Piperidino, N-Morpholino, unsubstituiertes oder durch Halogen, $C_1$-$C_{12}$-Alkyl oder $C_1$-$C_{12}$-Alkoxy substituiertes Phenyl, Phenoxy, Phenylmercapto, Phenoxycarbonyl oder Phenylcarbamoyl sind, und T die oben angegebene Bedeutung hat.

Bevorzugt als Zwischenprodukte sind Pyrrolopyrrole der Formel VIII, worin A und B gleich sind und eine der Gruppen

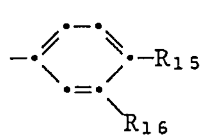 oder 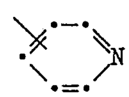

bedeuten, worin einer der Substituenten $R_{15}$ und $R_{16}$ ein Wasserstoff-, Chlor- oder Bromatom, eine Methyl-, Methoxy-, Methylmercapto-, Cyan-, Phenyl-, Phenoxy- oder Phenylmercaptogruppe ist und der andere ein Wasserstoffatom bedeutet, und

T Chlor oder eine Gruppe -O-G ist, worin

G eine der Gruppen
$-POCl_2$ oder $POClZ$,
worin Z Phenyl, Phenoxy oder Phenylamino ist, bedeutet.

Besonders bevorzugte Zwischenprodukte sind Pyrrolopyrrole der Formel VIII, worin A und B gleich sind und eine Gruppe

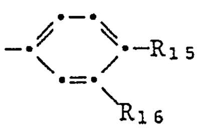

bedeuten, worin einer der Substituenten $R_{15}$ und $R_{16}$ ein Wasserstoff- oder Chloratom, eine Methyl-, Cyan- oder Phenylgruppe ist und der andere ein Wasserstoffatom bedeutet, und
T Chlor oder eine Gruppe $-O-POCl_2$ ist.

Die Pyrrolopyrrole der Formeln I und II, und unter denen der Formel I insbesondere diejenigen, worin X eine Gruppe -NRR′ bedeutet, eignen sich zum Färben von hochmolekularem organischem Material, insbesondere von synthetischen Polymeren, wie z.B. Polycarbonate, Polyacrylate, Polymethacrylate, ABS, Polyetherketone, Polyurethane, Polyolefine, Polyester und Polyamide, einzeln oder in Mischungen, und darunter vor allem die Ingenieurwerkstoffe (Engineering Plastics), wobei sie farbstarke brillante, transparente Ausfärbungen mit guten Allgemeinbeständigkeiten ergeben. Besonders bevorzugt ist die Einfärbung von Polyolefinen, Polyestern und Polyamiden. Sie können aber auch zum Einfärben von Lacken und Druckfarben verwendet werden.

Die Einfärbung erfolgt nach den üblichen Verfahren, beispielsweise durch Mischen des Farbmittels mit dem Kunststoffgranulat oder -pulver und Extrudieren der Mischung zu Fasern, Folien oder Granulaten. Letztere können dann im Spritzgussverfahren zu Gegenständen verformt werden.

Die erhaltenen Färbungen, beispielsweise in Kunststoffen, Fasern, Lacken oder Drucken, zeichnen sich durch einen gelben bis blauen Farbton, eine sehr grosse Farbstärke, hohe Sättigung, gute Dispergierbarkeit, gute Ueberlackier-, Migrations-, Hitze-, Licht- und Wetterechtheit sowie durch einen guten Glanz und gutes IR-Remissionsverhalten aus.

Die Verbindungen der Formeln I und II können auch als photoelektrophoretische Toner verwendet werden.

Wenn die Verbindungen der Formeln I und II in den angewandten Polymeren gelöst vorliegen, zeichnen sie sich ebenfalls durch reinen Farbton, grosse Farbstärke, gute Echtheiten, insbesondere Licht- und Sublimierechtheit, und ausserdem durch hohe Fluoreszenz aus. Sie eignen sich in Sonnenenergie-Kollektoren und zur Herstellung von Laser-Strahlen.

Die Pyrrolopyrrole der Formeln I und II, insbesondere aber diejenigen der Formel I, worin X eine Gruppe -SH bedeutet, haben, wie bereits erwähnt, auch eine photoleitende Wirkung. Sie können demnach als photoleitfähige Substanzen, beispielsweise in elektrophotographische Aufzeichnungsmaterialien, eingearbeitet werden. Der Aufbau solcher elektrophotographischer Aufzeichnungsmaterialien, die Einarbeitungsmöglichkeiten der photoleitfähigen Substanzen und ihre Wirkungsweise sind in EP-A-187 620 mit Bezug auf Dithioketopyrrolopyrrole beschrieben. Für die erfindungsgemässen Thioketo-pyrrolopyrrole gelten die gleichen Prinzipien.

Die nachfolgenden Beispiele dienen zur Erläuterung der Erfindung.

Beispiel 1:

In einem 750 ml Sulfierkolben mit Rührer, Thermometer, Rückflusskühler und Stickstoff-Spülung werden 28,8 g 1,4-Diketo-3,6-diphenylpyrrolo[3,4-c]pyrrol und 275 ml Phosphoroxychlorid vorgelegt. Nach Zugabe einer katalytischen Menge Dimethylformamid (~0,2 g) wird die Suspension auf Rückflusstemperatur (105-106°C) erhitzt und etwa 20 Stunden bei dieser Temperatur gerührt, wobei das Ausgangsmaterial in Lösung geht und das gebildete Produkt teilweise ausfällt. Das Reaktionsgemisch wird auf ~4°C mit einem Eisbad gekühlt und etwa 5 Stunden bei dieser Temperatur gerührt. Das kristalline Produkt wird schliesslich abfiltriert, mit Ether gewaschen und unter Feuchtigkeitsausschluss bei 80°C im Vakuumtrockenschrank getrocknet. Man erhält 38,6 g (87,5 % d.Th) eines roten Produkts der Formel

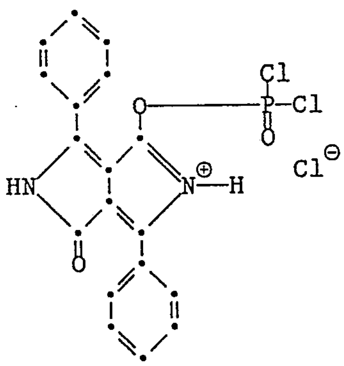

| Elementar-Analyse: | berechnet | gefunden |
|---|---|---|
| C | 48,95 % | 50,2 % |
| H | 2,74 % | 2,90 % |
| N | 6,34 % | 6,33 % |
| Cl | 24,1 % | 22,3 % |
| P | 7,01 % | 6,7 % |

Schmelzpunkt: 235-237°C (Zers.).

Beispiel 2:

4,51 g des Produktes von Beispiel 1 werden mit 40 ml kaltem Wasser in einem 200 ml Sulfierkolben mit Rührer unter Stickstoff vorgelegt. Die Suspension wird auf 2-4°C mit einem Eisbad gekühlt, und dann wird eine wässrige Natriumsulfidlösung aus 8,03 g $Na_2S{\bullet}8H_2O$ und 20 ml Wasser in der Weise zugegeben, dass der pH im Bereich zwischen 8,5 und 11 gehalten wird und die Temperatur spontan bis auf 20°C ansteigt. Die Natriumsulfidlösung wird wie oben beschrieben so lange zudosiert, bis keine Aenderung des pH mehr eintritt. Dabei wird nach dreistündigem Rühren bei 20°C das Reaktionsgemisch auf 32-34°C erwärmt und so lange bei dieser Temperatur gehalten, bis die Umsetzung beendet ist (insgesamt 7 Stunden). Die Suspension wird danach auf Raumtemperatur abgekühlt, filtriert und mit warmem Wasser neutral gewaschen. Das feuchte Produkt wird anschliessend in 100 ml Eisessig aufgeschlämmt und während 1 $\frac{1}{2}$ Stunden bei 80°C nachbehandelt, dann auf 45°C abgekühlt, abfiltriert und nacheinander mit Methanol und warmem Wasser neutral gewaschen. Nach dem Trocknen bei 80°C im Vakuumtrockenschrank erhält man 2,34 g (77 % d.Th.) einer Verbindung der Formel

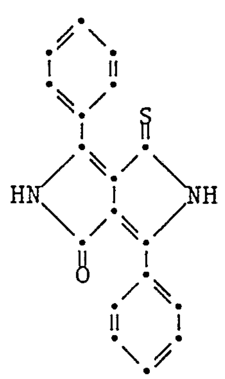

Durch Umkristallisieren aus Dimethylformamid erhält man ein violett-blaues Produkt mit einem Schmelzpunkt über 300°C und folgenden Analysewerten:

| | berechnet | gefunden |
|---|---|---|
| C | 71,03 % | 71,2 % |
| H | 3,97 % | 4,05 % |
| N | 9,20 % | 9,12 % |
| S | 10,53 % | 10,2 % |

Beispiel 3:

0,65 g des Produktes von Beipiel 1 und 0,57 g Anilin werden in 10 ml 1,1,2,2-Tetrachlorethan in einem Rundkolben 3 Stunden am Rückfluss gekocht. Nach dem Abkühlen wird das Reaktionsprodukt mit Aceton verdünnt, abgenutscht, mit Aceton und dann mit Wasser neutral gewaschen und schliesslich über eine $SiO_2$-Säule gereinigt (Eluiermittel: Methylenchlorid/Methanol, 95:5 Volumenteile). Man erhält 0,22 g einer Verbindung der Formel

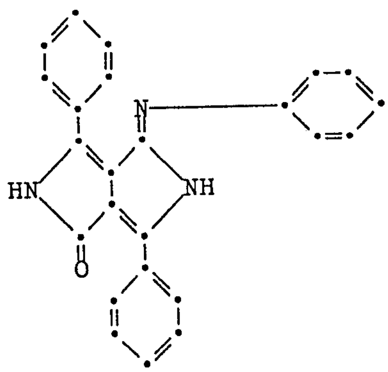

| Elementar-Analyse: | berechnet | gefunden |
|---|---|---|
| C | 79,3 % | 78,5 % |
| H | 4,7 % | 4,8 % |
| N | 11,6 % | 11,4 % |

Beispiel 4:

Ein Gemisch bestehend aus 2,88 g 1,4-Diketo-3,6-diphenylpyrrolo[3,4-c]pyrrol, 4,7 g Phosphoroxychlorid und 25 ml Tetrahydrothiophen-1,1-dioxyd (Sulfolan) wird in einem 100 ml Sulfierkolben unter Argon vorgelegt. Es werden noch 2 Tropfen Dimethylacetamid (~90 mg) als Katalysator zugesetzt. Das Gemisch wird auf 120°C erhitzt und 2 Stunden bei dieser Temperatur gehalten. Das Ausgangspigment geht dabei in Lösung. Nach dem Abkühlen auf 115°C wird unter vermindertem Druck (~200 mbar) das unverbrauchte Phosphoroxychlorid (~1,5 ml) abdestilliert. Unter Spülung mit Argon wird danach der Kolbeninhalt auf ca 80°C abgekühlt und mit 1,46 g 4-Aminobenzonitril versetzt. Man heizt wieder auf 120°C und hält 2 Stunden bei dieser Temperatur. Anschliessend wird die Suspension auf 30°C abgekühlt, in eine eiskalte 5 %ige wässrige Ammoniak-Lösung gegossen und 15 Minuten verrührt. Das ausgefallene Produkt wird dann abfiltriert und mit Wasser gewaschen, bis das Filtrat farblos und neutral abläuft. Nach der Trocknung im Vakuumtrockenschrank bei 80°C erhält man 3,9 g (100 % d.Th.) Rohprodukt. Nach Reinigung in kochendem Methanol am Rückfluss isoliert man 2,75 g eines violetten Produktes der Formel

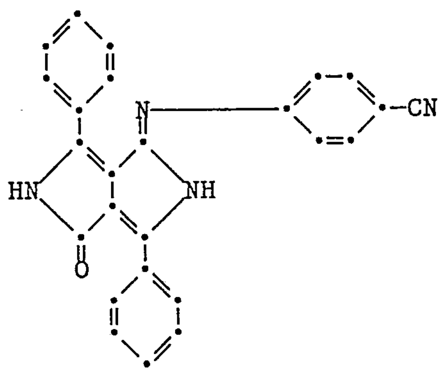

,

mit Schmelzpunkt über 300°C und folgenden Analysenwerten:

| | berechnet | gefunden |
|---|---|---|
| C | 77,30 % | 76,70 % |
| H | 4,15 % | 4,25 % |
| N | 14,42 % | 14,65 % |

Beispiel 5:

Wiederholt man das Beispiel 4 mit der einzigen Ausnahme, dass man das 4-Aminobenzonitril durch 1,7 g 4-Nitroanilin ersetzt, so isoliert man bei der Fällung durch Eingiessen des Reaktionsgemisches in eine 5 %-ige wässrige Ammoniak-Lösung 3,7 g (91,4 % d.Th.) Rohprodukt. Hach Reinigung durch Kochen in einer Mischung von Methylenchlorid und Methanol (4:1 Volumenteile) am Rückfluss erhält man 2,5 g eines dunkelblauen Produktes der Formel

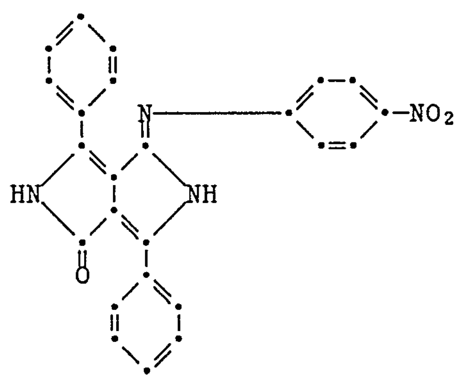

mit Schmelzpunkt über 300°C und folgenden Analysenwerten:

| | berechnet | gefunden |
|---|---|---|
| C | 70,58 % | 69,9 % |
| H | 3,95 % | 3,8 % |
| N | 13,72 % | 13,5 % |

Beispiel 6:

Wiederholt man das Beispiel 4 mit dem einzigen Unterschied, dass man das 4-Aminobenzonitril durch 1,55 g 4-Chloranilin ersetzt, so isoliert man bei der Fällung durch Eingiessen des Reaktionsgemisches in eine 5 %-ige wässrige Ammoniak-Lösung 3,4 g (85,4 % d.Th.) Rohprodukt. Nach Reinigung in kochendem Methanol am Rückfluss erhält man 2,5 g eines rotblauen Produktes der Formel

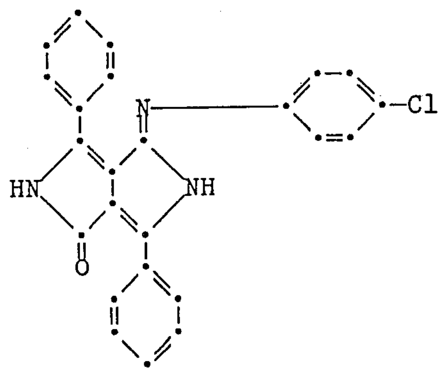

mit Schmelzpunkt über 300°C und folgenden Analysenwerten:

| | berechnet | gefunden |
|---|---|---|
| C | 72,45 % | 72,0 % |
| H | 4,05 % | 4,08 % |
| N | 10,56 % | 10,25 % |
| Cl | 8,91 % | 8,75 % |

Beispiel 7:

Ein Gemisch aus 5,76 g 1,4-Diketo-3,6-diphenylpyrrolo[3,4-c]pyrrol, 4,64 g Phosphoroxychlorid und 50 ml Tetrahydrothiophen-1,1-dioxyd (Sulfolan) wird in einem 200 ml Sulfierkolben unter Stickstoff vorgelegt, dann werden noch 4 Tropfen Dimethylacetamid (~180 mg) als Katalysator zugesetzt. Das Gemisch wird auf 118°C erwärmt, 13 Stunden bei dieser Temperatur gehalten und anschliessend auf Raumtemperatur abgekühlt. Das unreagierte Ausgangsmaterial wird abfiltriert und mit wenig warmem Sulfolan gewaschen. Danach wird das Filtrat zur vollständigen Entfernung von noch vorhandenem Phosphoroxychlorid 1 $\frac{1}{2}$ Stunden im Rotationsverdampfer bei 80°C unter Vakuum behandelt.

Die zurückgebliebene Lösung wird in einem 200 ml Sulfierkolben unter Stickstoff vorgelegt, dann werden 8,32 g technischer Anthranilsäureethylester (~95 %ig) zugefügt. Das Gemisch wird auf 87°C geheizt und 8 Stunden bei dieser Temperatur gehalten. Nach dem Abkühlen auf Raumtemperatur wird die Reaktionslösung unter kräftigem Rühren auf 400 ml kaltes Wasser gegossen, wobei das Rohprodukt ausfällt. Die stark saure Suspension wird mit Triethanolamin auf pH 7 gestellt, auf 50°C erwärmt, 1 Stunde gerührt und filtriert. Das Nutschgut wird mit Wasser und Ethanol gewaschen und bei 80°C im Vakuumtrockenschrank getrocknet. Man erhält 3,74 g eines violetten Produktes der Formel

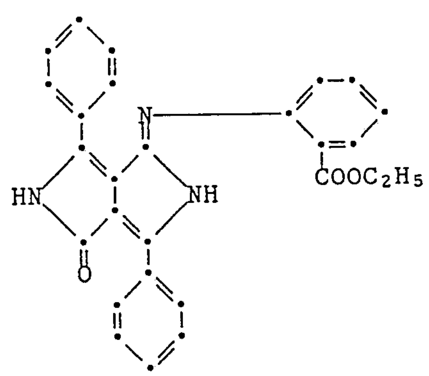

mit Schmelzpunkt ~235°C (darüber wird die Probe wieder fest) und folgenden Analysenwerten:

| | berechnet | gefunden |
|---|---|---|
| C | 74,47 % | 73,30 % |
| H | 4,86 % | 4,82 % |
| N | 9,65 % | 9,55 % |

Beispiel 8:

2,22 g des Produktes aus Beispiel 7 werden zusammen mit 2 ml Eisessig und 25 ml reinem o-Dichlorbenzol

in einem 100 ml Sulfierkolben vorgelegt und kräftig gerührt. Die erhaltene Suspension wird auf 125° C geheizt und 4 ½ Stunden bei dieser Temperatur weitergerührt. Dabei geht vorerst das Ausgangsmaterial in Lösung und dann fällt das cyclisierte Produkt aus. Das Reaktionsgemisch wird auf 50° C abgekühlt, das kristalline Produkt wird abfiltriert, mit Aceton und Methanol gewaschen, bis die Auswaschlösung farblos abläuft, und im Vakuumtrockenschrank bei 80° C getrocknet. Man isoliert 1,41 g eines einheitlichen violetten Produktes der Formel

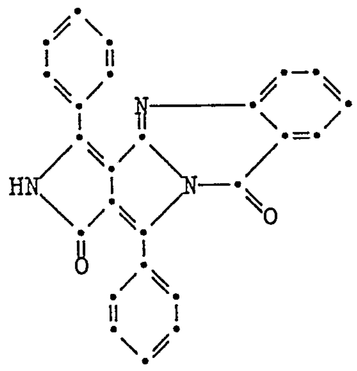

mit Schmelzpunkt über 300° C und folgenden Analysenwerten:

| | berechnet | gefunden |
|---|---|---|
| C | 77,11 % | 76,60 % |
| H | 3,88 % | 3,99 % |
| N | 10,79 % | 10,73 % |

Beispiel 9:

Analog Beispiel 1 werden 10 g 1,4-Diketo-3,6-(3,4-dimethylphenyl)-pyrrolo[3,4-c]-pyrrol in 133 g Phosphoroxychlorid in Gegenwart von 4 Tropfen Dimethylformamid behandelt. Man isoliert 6,6 g dunkelrotes Pulver. Analog Beispiel 3 werden 2,0 g dieses Produktes in 15 ml Ethylenchlorid mit 0,41 g Anilin in Gegenwart von 0,45 g Triethylamin behandelt. Man isoliert 1,50 g (89 %) eines dunkelroten Produktes der Formel

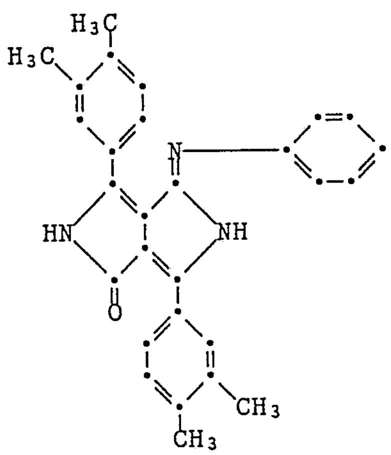

dessen Konstitution durch das Massenspektrum (MS) bestätigt wird (MS: $M^+ = 491$).

Beispiel 10:

1,0 g des Produktes aus Beispiel 1 wird in 10 ml Ethylenchlorid mit 0,40 g Mercaptobenzthiazol und 0,25 g Triethylamin 20 Std. unter Rückfluss erhitzt. Nach dem Abkühlen, der Filtration und dem Waschen mit Aceton isoliert man 0,76 g eines Gemisches aus 1,4-Diketo-3,6-diphenyl-pyrrolo[3,4-c]-pyrrol und einem Produkt der Formel

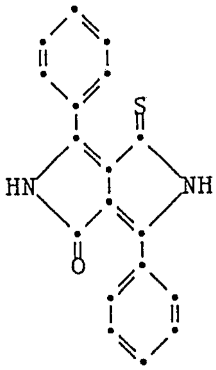

im Verhältnis ca. 2:1 (Bestätigt durch MS, $^1$H-NMR).

Beispiel 11:

10.16 g des Produktes aus Beispiel 1 und 4.60 g p-Chloranthranilsäureethylester werden unter Feuchtigkeitsausschluss in 300 ml o-Dichlorbenzol 18 Stunden bei 75°C gerührt. Das Reaktionsgemisch wird auf Raumtemperatur gekühlt und 3,2 ml Triethylamin zugegeben. Das Reaktionsgemisch wird in 500 ml eines 1:1 Gemisches von Toluol und Essigsäureethylester gegossen und 2 Stunden bei 80°C gerührt. Nach dem Abkühlen auf Raumtemperatur wird filtriert und der Rückstand mit 500 ml eines 1:1 Gemisches von Toluol und Essigsäureethylester gewaschen. Nach dem Trocknen des Rückstandes im Vakuumtrockenschrank erhält man 12,60 g Festprodukt. Durch Filtration an wenig Kieselgel mit MethylenchloridMethanol 1:1 wird ein unlösliches Nebenprodukt abgetrennt und man erhält nach dem Eindampfen 7,3 g eines Gemisches der Produkte der Formeln

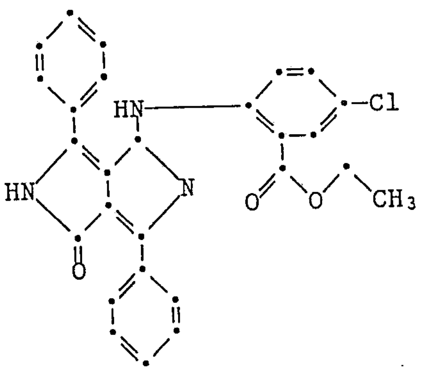

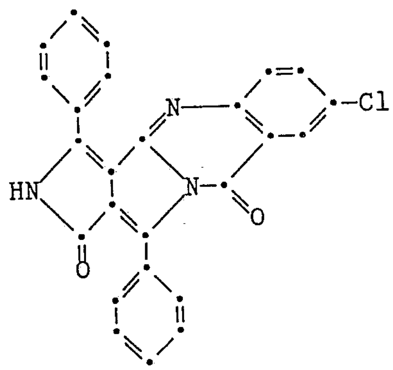

(Massenspektrum: $M^+ = 469$ und $M^+ = 423$).

Beispiel 12:

5 g des Gemisches aus Beispiel 11 werden in 400 ml o-Dichlorbenzol suspendiert, mit 20 ml Essigsäure versetzt und 22 Stunden auf 140°C erwärmt. Nach dem Abkühlen auf Raumtemperatur wird filtriert und mit 400 ml o-Dichlorbenzol und 500 ml Methylenchlorid gewaschen. Trocknen im Vakuumtrockenschrank liefert 2,24 g Produkt der Formel

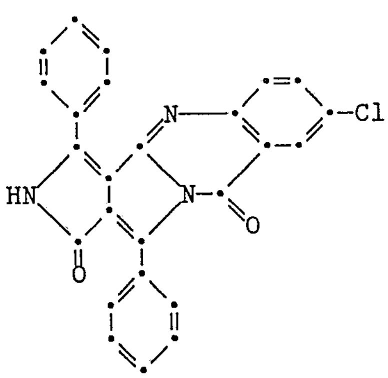

dessen Konstitution durch MS ($M^+ = 423$) bestätigt wird.

| Elementaranalyse: | berechnet | gefunden |
| --- | --- | --- |
| C | 70,84 | 69,05 |
| H | 3,33 | 3,63 |
| N | 9,91 | 9,36 |
| Cl | 8,36 | 8,94 |

Beispiel 13:

100 g eines bei 110°C unter Hochvakuum getrockneten Polyestergranulats (TERLENKA®, der Firma Enka) werden mit 0,2 g des Produktes von Beispiel 8 während 30 Minuten auf einer Schüttelmaschine vermischt. Das erhaltene, gleichmässig gefärbte Granulat wird dann in einem Laborextruder des Typs Weber ET 20® bei 260°C zu einem dünnen durchsichtigen, fluoreszierenden, roten Band extrudiert. Die Ausfärbung zeichnet sich durch einen farbstarken, reinen Farbton und durch gute Allgemeinbeständigkeiten aus.

Eine Ausfärbung mit ähnlichen Eigenschaften erhält man, wenn das Produkt von Beispiel 8 durch das Produkt von Beispiel 7 ersetzt wird.

Beispiel 14:

(Anwendung im Alkyd-Melamin-Einbrennlack) In einer 100 ml Schraubdeckelflasche werden 135 g Glasperlen (4,5 mm), 400 mg Produkt aus dem Beispiel 8, 7,6 g $TiO_2$ (®,Bayertitan R-KB-3), 9,0 ml Methylisobutylketon und 30,0 g Alkydmelamin-Einbrennlack der Zusammensetzung

| | |
| --- | --- |
| 67,5 % | Alkydharz (®Alkydal F-27), 60 % in Xylol, |
| 26,5 % | Melaminharz (®Maprenal TTX), 55 % in Xylol, |
| 5,0 % | Xylol und |
| 1,0 % | Siliconöl, 1 % in Xylol |

(Festkörperanteil = 55 %) gegeben und 16 Std. auf der Schwingmühle gemahlen. Der eingefärbte Lack wird mit einem Filmziehgerät auf einem Aluminium-Streifen ausgezogen. Nach dem Ablüften wird der Lackstreifen während 30 Min. bei 130°C eingebrannt. Man erhält eine violette Lackierung von guter Licht- und Wetterechtheit.

**Patentansprüche**

1. Pyrrolopyrrole der Formeln

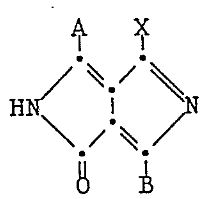

(I)

oder

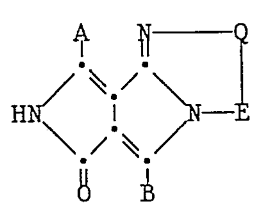

(II),

worin A und B unabhängig voneinander carbocyclische oder heterocyclische aromatische Reste, acyclische oder cyclische aliphatische Reste oder araliphatische Reste sind, Q einen zweiwertigen carbocyclischen oder heterocyclischen aromatischen, acyclischen oder cyclischen aliphatischen oder araliphatischen Rest bedeutet, E eine direkte Bindung oder eine Gruppe -CO- oder -CS- ist, X eine Gruppe -SR oder -NRR′ bedeutet, worin R und R′ unabhängig voneinander Wasserstoff, carbocyclische

oder heterocyclische aromatische Reste, acyclische oder cyclische aliphatische Reste oder araliphatische Reste sind und R′ zusätzlich eine Gruppe -Q-Halogen oder -Q-EY-($C_1$-$C_5$-Alkyl), worin Y O oder S bedeutet, sein kann, oder R und R′ mit dem N-Atom, an dem sie gebunden sind, einen 5- oder 6-gliedrigen heterocyclischen Rest bilden.

2. Pyrrolopyrrole der Formel II, gemäss Anspruch 1, worin Q, wenn E -CO-oder -CS- bedeutet, eine Gruppe der Formeln

$-C(R_7)(R_8)-$, (III) $-C(R_9)(R_{10})-C(R_{11})(R_{12})-$ (IV) oder $-CR_{13}=CR_{14}-$ (V)

und, wenn E eine direkte Bindung bedeutet, eine Gruppe der Formeln IV oder V ist, wobei in den Formeln III bis V $R_7$ und $R_8$ unabhängig voneinander Wasserstoff, $C_1$-$C_5$-Alkyl, $C_5$-$C_8$-Cycloalkyl, unsubstituiertes oder durch Halogen, Hydroxy, $C_1$-$C_5$-Alkyl oder $C_1$-$C_5$-Alkoxy oder Phenoxy substituiertes Phenyl oder Benzyl oder eine der Gruppen

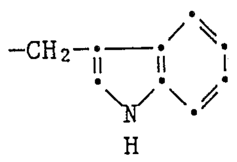 , 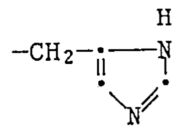

oder $-(CH_2)_2-S(C_1$-$C_5$-Alkyl) bedeuten, $R_9$, $R_{10}$, $R_{11}$ und $R_{12}$ unabhängig voneinander Wasserstoff, $C_1$-$C_4$-Alkyl, $C_5$-$C_6$-Cycloalkyl, unsubstituiertes oder durch Halogen, Hydroxy, $C_1$-$C_5$-Alkyl oder $C_1$-$C_5$-Alkoxy substituiertes Phenyl oder Benzyl bedeuten oder $R_{10}$ und $R_{12}$ zusammen mit den C-Atomen, an denen sie gebunden sind, eine $C_5$-$C_6$-Cycloalkylengruppe bilden, und $R_{13}$ und $R_{14}$ unabhängig voneinander Wasserstoff, $C_1$-$C_5$-Alkyl, $C_5$-$C_6$-Cycloalkyl, unsubstituiertes oder durch Halogen, Hydroxy, $C_1$-$C_5$-Alkyl oder $C_1$-$C_5$-Alkoxy substituiertes Phenyl oder Benzyl bedeuten oder $R_{13}$ und $R_{14}$ zusammen mit den C-Atomen, an denen sie gebunden sind, einen Naphthalin-, Anthracen- oder Phenanthrenring oder eine der Gruppen

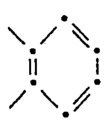 , 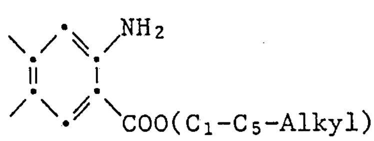 , 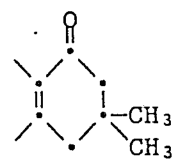 , 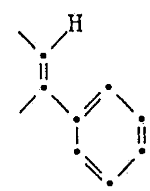 oder

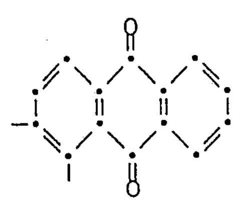 bilden.

3. Pyrrolopyrrole der Formeln I oder II gemäss Anspruch 1, worin A und B unabhängig voneinander eine der Gruppen

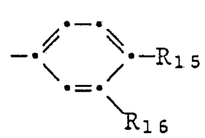 oder 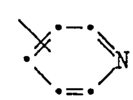

bedeuten, worin $R_{15}$ und $R_{16}$ unabhängig voneinander Wasserstoff, Halogen, Nitro, Carbamoyl, Cyano, Trifluormethyl, $C_2$-$C_{13}$-Alkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxycarbonyl, $C_2$-$C_{13}$-Alkanoylamino, $C_1$-$C_{12}$-Monoalkylamino, $C_2$-$C_{24}$-Dialkylamino, Mono-($C_5$-$C_7$-cycloalkyl)-amino, Di-($C_5$-$C_7$-cycloalkyl)-amino, H-Pyrrolidino, N-Piperidino, N-Morpholino, unsubstituiertes oder durch Halogen, $C_1$-$C_{12}$-Alkyl oder $C_1$-$C_{12}$-Alkoxy substituiertes Phenyl, Phenoxy, Phenylmercapto, Phenoxycarbonyl oder Phenylcarbamoyl sind,X eine Gruppe -SR oder -NRR′ bedeutet,
R Wasserstoff, $C_1$-$C_{12}$-Alkyl, Cyclohexyl, β-Methoxyallyl, Benzyl, unsubstituiertes oder durch Halogen, Cyano, Nitro, $C_1$-$C_5$-Alkyl, $C_1$-$C_5$-Alkoxy, ($C_1$-$C_5$-Alkoxy)-carbonyl, $C_1$-$C_5$-Alkylmercapto, Phenylmercapto oder Phenoxy substituiertes Phenyl ist, oder R ferner Pyridyl, Pyrimidyl, Pyrazinyl, Triazinyl, Furyl, Pyrrolyl, Thienyl, Chinolinyl, Cumarinyl, Benzofuryl, Benzimidazolyl oder Benzoxazolyl bedeutet,
R′ $C_1$-$C_{12}$-Alkyl, β-Dimethylaminoethyl, Cyclohexyl, Cyanethyl, β-Ethoxycarbonylethyl, Benzyl, 1-Phenyl-2-ethoxycarbonyl-vinyl, unsubstituiertes oder durch Halogen, Cyano, Nitro, $C_1$-$C_5$-Alkyl, $C_1$-$C_5$-Alkoxy, ($C_1$-$C_5$-Alkoxy)-carbonyl, $C_1$-$C_5$-Alkylmercapto, Phenoxy oder Phenylmercapto substituiertes Phenyl ist, oder R′ ferner Pyridyl, Pyrimidyl, Chinolinyl, Pyrazinyl, Triazinyl, Furyl, Pyrrolyl, Thienyl, Cumarinyl, Benzofuryl, Anthrachinonyl, Benzimidazolyl oder Benzoxazolyl ist oder eine Gruppe
-Q-EY-($C_1$-$C_5$-Alkyl),
bedeutet, oder R und R′ zusammen mit dem N-Atom einen Piperidin-, 2,2,6,6-Tetramethylpiperidin-, Pyrrol-, Morpholin-, Pyrrolidin- oder Phthalimidring bilden,
E und Y die in Anspruch 1 angegebene Bedeutung haben, und
Q unsubstituiertes oder durch Halogen, Cyano, Nitro, $C_1$-$C_5$-Alkyl, $C_1$-$C_5$-Alkoxy oder ($C_1$-$C_5$-Alkoxy)-carbonyl substituiertes o-Phenylen oder eine zweiwertige Gruppe der Formel
-$C(R_7)(R_8)$- oder -$CR_{13}=CR_{14}$- bedeutet,
$R_7$ und $R_8$ unabhängig voneinander Wasserstoff, $C_1$-$C_5$-Alkyl oder Phenyl sind und
$R_{13}$ und $R_{14}$ unabhängig voneinander Wasserstoff, $C_1$-$C_4$-Alkyl oder Phenyl sind oder zusammen mit den C-Atomen an denen sie gebunden sind einen Naphthalin-, Anthracen- oder Phenanthrenring bilden.

4. Pyrrolopyrrole der Formeln I oder II gemäss Anspruch 1, worin A und B gleich sind und eine der Gruppen

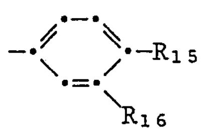

oder

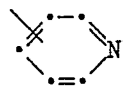

bedeuten, worin einer der Substituenten $R_{15}$ und $R_{16}$ ein Wasserstoff-, Chlor- oder Bromatom, eine Methyl-, Methoxy-, Methylmercapto-, Cyan-, Phenyl-, Phenoxy- oder Phenylmercaptogruppe ist und der andere ein Wasserstoffatom bedeutet,
X eine Gruppe -SH oder -NHR′ bedeutet,
R′ $C_1$-$C_{12}$-Alkyl, unsubstituiertes oder durch Halogen, Cyano, Nitro, Methyl, Methoxy, Methylmercapto, Methoxy- oder Ethoxycarbonyl substituiertes Phenyl ist oder eine Gruppe -Q-EY($C_1$-$C_5$-Alkyl) bedeutet,
E und Y die in Anspruch 1 angegebene Bedeutungen haben und
Q o-Phenylen oder eine zweiwertige Gruppe der Formel
-$CHR_8$- oder -$CR_{13}=CR_{14}$-
bedeutet,
$R_8$ Wasserstoff, Methyl oder Phenyl bedeutet und
$R_{13}$ und $R_{14}$ Wasserstoff, Methyl oder Phenyl sind.

5. Pyrrolopyrrole der Formeln I oder II gemäss Anspruch 1, worin A und B gleich sind und eine Gruppe

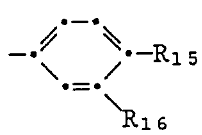

bedeuten, worin einer der Substituenten $R_{15}$ und $R_{16}$ ein Wasserstoff- oder Chloratom, eine Methyl-, Cyan- oder Phenylgruppe ist und der andere ein Wasserstoffatom bedeutet,
X eine Gruppe -SH oder -NHR′ bedeutet,
R′ unsubstituiertes oder durch Chlor, Brom, Methyl, Cyano, Nitro, Methoxy, Methylmercapto, Methoxy- oder Ethoxycarbonyl substituiertes Phenyl ist,
E die in Anspruch 1 angegebene Bedeutung hat und
Q o-Phenylen bedeutet.

6. Pyrrolopyrrole der Formel

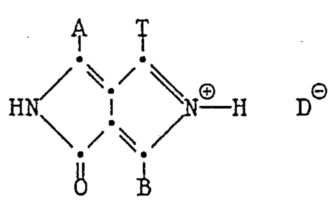

(VIII),

worin A und B unabhängig voneinander carbocyclische oder heterocyclische aromatische Reste, acyclische oder cyclische aliphatische Reste oder araliphatische Reste sind, und T Chlor, Brom oder eine Gruppe -O-G bedeutet, worin G eine Gruppe -SOCl, -SOBr, -$POCl_2$, -POClBr, -$POBr_2$, -POClZ oder -POBrZ bedeutet und Z Phenyl, Phenoxy, Phenylmercapto, Di-($C_1$-$C_4$-alkyl)-amino, Phenylamino, Diphenylamino oder Phenyl-($C_1$-$C_4$-alkyl)-amino ist, wobei die Phenylgruppen auch durch Halogen oder $C_1$-$C_5$-Alkyl substituiert sein können.

7. Pyrrolopyrrole der Formel VIII gemäss Anspruch 6, worin A und B unabhängig voneinander eine der

Gruppen

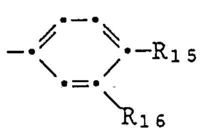 oder 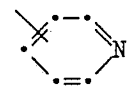

bedeuten, worin $R_{15}$ und $R_{16}$ unabhängig voneinander Wasserstoff, Halogen, Nitro, Carbamoyl, Cyano, Trifluormethyl, $C_2$-$C_{13}$-Alkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxycarbonyl, $C_2$-$C_{13}$-Alkanoylamino, $C_1$-$C_{12}$-Monoalkylamino, $C_2$-$C_{24}$-Dialkylamino, Mono-($C_5$-$C_7$-cycloalkyl)-amino, Di-($C_5$-$C_7$-cycloalkyl)-amino, N-Pyrrolidino, N-Piperidino, N-Morpholino, unsubstituiertes oder durch Halogen, $C_1$-$C_{12}$-Alkyl oder $C_1$-$C_{12}$-Alkoxy substituiertes Phenyl, Phenoxy, Phenylmercapto, Phenoxycarbonyl oder Phenylcarbamoyl sind, und T die in Anspruch 6 angegebene Bedeutung hat.

8. Pyrrolopyrrole der Formel VIII gemäss Anspruch 6, worin A und B gleich sind und eine der Gruppen

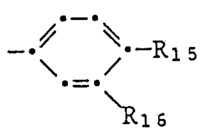 oder 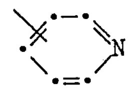

bedeuten, worin einer der Substituenten $R_{15}$ und $R_{16}$ ein Wasserstoff-, Chlor- oder Bromatom, eine Methyl-, Methoxy-, Methylmercapto-, Cyan-, Phenyl-, Phenoxy- oder Phenylmercaptogruppe ist und der andere ein Wasserstoffatom bedeutet, und
T Chlor oder eine Gruppe -O-G ist, worin
G eine der Gruppen
-$POCl_2$ oder POClZ,
worin Z Phenyl, Phenoxy oder Phenylamino ist, bedeutet.

9. Pyrrolopyrrole der Formel VIII gemäss Anspruch 6, worin A und B gleich sind und eine Gruppe

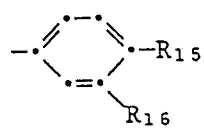

bedeuten, worin einer der Substituenten $R_{15}$ und $R_{16}$ ein Wasserstoff- oder Chloratom, eine Methyl-, Cyan- oder Phenylgruppe ist und der andere ein Wasserstoffatom bedeutet, und
T Chlor oder eine Gruppe -O-$POCl_2$ ist.

10. Stoffzusammensetzung enthaltend ein hochmolekulares organisches Material und als Färbemittel ein Pyrrolopyrrol der Formeln I oder II gemäss Anspruch 1.

11. Stoffzusammensetzung gemäss Anspruch 10 enthaltend ein synthetisches Polymer und als Färbemittel ein Pyrrolopyrrol der Formel I, worin X eine Gruppe -NRR′ bedeutet, oder der Formel II.

12. Elektrophotographisches Aufzeichnungsmaterial enthaltend als photoleitfähige Substanz ein Pyrrolopyrrol der Formeln I oder II gemäss Anspruch 1.

13. Elektrophotographisches Aufzeichnungsmaterial gemäss Anspruch 12 enthaltend als photoleitfähige Substanz ein Pyrrolopyrrol der Formel I, worin X eine Gruppe -SR bedeutet.

Europäisches Patentamt

# EUROPÄISCHER RECHERCHENBERICHT

Nummer der Anmeldung

EP 89810253.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A2/A3 - 0 224 445 (CIBA-GEIGY) * Ansprüche * -- | 1,3-5, 10 | C 07 D 487/04<br>C 07 D 487/14<br>C 09 B 57/00<br>G 03 G 5/06<br>//(C 07 D 487/04<br>C 07 D 209:00<br>C 07 D 209:00)<br>(C 07 D 487/14<br>C 07 D 209:00<br>C 07 D 209:00<br>C 07 D 239:00) |
| D | EP - A2/A3 - 0 232 222 (CIBA-GEIGY) | | |
| X | * Ansprüche 1,2,4-9 * | 1,3-5 | |
| A | * Ansprüche; Seite 7, Absatz 1 * -- | 6-10 | |
| A | EP - A2/A3 - 0 181 290 (CIBA-GEIGY) * Ansprüche * -- | 1,3-6, 10,11 | |
| A | EP - A2/A3 - 0 133 156 (CIBA-GEIGY) * Ansprüche * -- | 1,3-6, 10,11 | |
| D,A | US - A - 4 415 685 (IQBAL et al.) * Zusammenfassung; Ansprüche * -- | 1,3-6, 10,11 | RECHERCHIERTE SACHGEBIETE (Int. Cl.4) |
| A | EP - A2/A3 - 0 187 620 (CIBA-GEIGY) * Zusammenfassung; Ansprüche * ---- | 1,3-6, 12,13 | C 07 D 487/00<br>C 09 B 57/00 |

Der vorliegende Recherchenbericht wurde für alle Patentansprüche erstellt.

| Recherchenort | Abschlußdatum der Recherche | Prüfer |
|---|---|---|
| WIEN | 12-07-1989 | JANISCH |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument